# EUROPÄISCHE PATENTSCHRIFT

(11) **EP 1 423 160 B1**
(45) Veröffentlichungstag und Bekanntmachung des Hinweises auf die Patenterteilung: **06.02.2008**
(21) Anmeldenummer: 02797654.7
(22) Anmeldetag: 30.08.2002
(51) Int. Cl.: A61M 25/00

(54) **KATHETER UND VERFAHREN ZU SEINER HERSTELLUNG**
CATHETER AND METHOD FOR PRODUCING THE SAME
CATHETER ET SON PROCEDE DE PRODUCTION

(30) Priorität: 30.08.2001 SE 0102879
(43) Veröffentlichungstag der Anmeldung: 02.06.2004
(73) Patentinhaber: Roche Diagnostics GmbH, 68305 Mannheim (DE); Gambro Lundia AB, 220 10 Lund (SE); F.HOFFMANN-LA ROCHE AG, 4070 Basel (CH)
(72) Erfinder: HARTTIG, Herbert, 67122 Altrip (DE); BUCK, Reinhold, 88422 Alleshausen (DE)
(74) Vertreter: Knauer, Martin
(86) Internationale Anmeldenummer: PCT/EP2002/009686
(87) Internationale Veröffentlichungsnummer: WO 2003/020352

(56) Entgegenhaltungen:
- WO-A-96/17673
- WO-A-99/41606

## Beschreibung

Die vorliegende Erfindung betrifft einen Katheter nach dem Oberbegriff des Anspruchs 1 sowie ein Verfahren zur Herstellung eines derartigen Katheters nach dem Oberbegriff des Anspruchs 16.

Ein gattungsgemäßer Katheter für die Mikrodialyse ist aus der WO 99/41606 bekannt. Dieser Katheter weist je einen Zuführkanal und einen Abführkanal aus einer semipermeablen Membran für eine Dialyseflüssigkeit auf, welche in einem Mikrochip angeordnet sind. Beide Kanäle sind durch einen haarnadelförmigen Bereich miteinander verbunden, welcher aus dem Mikrochip herausragt und über welchen der Stoffaustausch zwischen Dialyse- und Körperflüssigkeit stattfindet. Der Abführkanal ist mit einer ebenfalls in den Mikrochip eingebetteten Analyseeinheit zur Überwachung der Zusammensetzung der Dialyseflüssigkeit nach dem Stoffaustausch integral verbunden. Diese Analyseeinheit setzt sich zusammen aus einem Reservoir mit einer Referenzsubstanz, einer Referenzelektrode oder ionensensitiven Elektrode und einem Sensor, bspw. einem ionensensitiven Feldeffekttransistor (ISFET). Problematisch bei dieser Ausführungsform, die lediglich eine Hohlfasermembran aufweist, ist, dass sie nur durch aufwendige chirurgische Maßnahmen ins Gewebe implantiert werden kann und zur Explantation ebenfalls wieder chirurgisch entfernt werden muss, da ein bloßes Herausziehen die Gefahr mit sich bringt, dass die Membran zumindest teilweise im Körper bzw. im Gewebe zurückbleibt.

Ein weiterer Katheter wird in der WO 98/44978 beschrieben. Dieser Katheter weist einen inneren schlauchförmigen Teil und einen diesen umgebenden äußeren schlauchförmigen Teil in Form einer Hohlfasermembran auf. Der innere schlauchförmige Teil umschließt einen Abführkanal. Die Außenwandung des inneren schlauchförmigen Teils und die Innenwandung des äußeren schlauchförmigen Teils begrenzen einen Zuführkanal. Ferner sind Distanzmittel vorgesehen, welche die Innenwandung des äußeren schlauchförmigen Teiles gegenüber der Außenwandung des inneren schlauchförmigen Teiles abstützen. Die Montage eines derartigen Katheters ist allerdings sehr kompliziert, weil die Einführung des inneren schlauchförmigen Teils mit den Distanzmitteln in den äußeren schlauchförmigen Teil weitaus schwieriger ist als bei einem glatten Schlauch.

Ein anderer Katheter zur Mikrodialyse ist in der DE 33 42 170 C2 offenbart. Dieser Katheter weist eine in einem Metallgehäuse aufgenommene rohrförmige oder schlauchförmige Dialysemembran, bspw. eine Hohlfasermembran, auf. Die Fertigung eines derartigen Katheters ist allerdings aufwendig, weil bei den geforderten Dimensionen ein sehr feiner Schlauch sehr genau in ein Gehäuse eingeführt werden muss. Außerdem neigt der Schlauch dazu, sich an die innere Gehäusewand anzulehnen und damit einen Teil der Membran zu blockieren, wodurch unvorhersehbare Austauschleistungen erbracht werden.

Bei allen bekannten Kathetern, bei denen konzentrische Rohre ineinander geschoben sind, ist es problematisch, dass die fluidische Kontaktierung, speziell des Spaltzwischenraumes zwischen dem äußeren und dem inneren Rohr bzw. der Hohlfasermembran und dem inneren Rohr, aufwendig ist und relativ viel Totvolumen erfordert. Totvolumina führen zu verlängerten Zeitspannen (Totzeit) zwischen einer Konzentrationsänderung und der Verfügbarkeit einer entsprechenden Probe in einer Messeinrichtung und sind daher zu minimieren. Da sich im Innern der Hohlfasermembran meistens die für die Trennung entscheidenden feinporigen Schichten befinden, besteht außerdem immer die Gefahr, dass beim Einschieben von Teilen in die Hohlfasermembran diese Trennschicht beschädigt wird.

Die Aufgabe der vorliegenden Erfindung besteht somit darin, einen Katheter der oben genannten Art bereit zu stellen, welcher unkompliziert herzustellen ist und einfach implantiert und explantiert werden kann.

Die Lösung besteht in einem Katheter mit den Merkmalen des Anspruchs 1 sowie in einem Verfahren mit den Merkmalen des Anspruchs 16.

Der erfindungsgemäße Katheter weist also eine um etwa 180°, d.h. etwa U-förmig gebogene Hohlfasermembran auf, wobei die beiden Schenkel des U möglichst eng beieinander liegen. Normalerweise würde dies dazu führen, dass die Hohlfasermembran abknickt, wodurch der Flüssigkeitsdurchtritt durch das Lumen unterbrochen würde. Überraschenderweise hat sich heraus gestellt, dass durch anisotropen Einsatz von Wärme oder Lösemitteln zumindest in diesem Bereich die Struktur der Hohlfasermembran so verändert wird, dass die Hohlfasermembran um etwa 180° gebogen werden kann, ohne abzuknicken, d.h. ohne dass sich das Lumen dabei verschließt.

Nachfolgende Untersuchungen haben ergeben, dass dies auf die anisotrope Einwirkung von Wärme oder Lösungsmitteln zurückführen ist. Durch die Einwirkung von Wärme oder Lösungsmittel wird das Membranmaterial weich. Die Oberflächen- bzw. Grenzflächenspannung des Membranmaterials bewirkt ein Schrumpfen der Poren. Dies äussert sich makroskopisch in einer Dimensionsänderung, d.h. die Membran wird kürzer, dünner und dichter. Da die Wärme oder die Lösemittel anisotrop, d.h von einer Seite her einwirken, kommt es zur Krümmung der Hohlfasermembran. Die Rückstellkräfte des von Wärme- oder Lösemitteleinwirkung nicht betroffenen Teils der Membran im Beigebereich werden durch von aussen wirkende mechanische Kräfte überwunden, um eine Biegung um 180 Grad zu erreichen. Beim Biegen um 180 Grad wird das Lumen zwar verengt, bleibt aber durchgängig.

Weiterhin ist es vorteilhaft, dass die Anschlüsse des erfindungsgemäßen Katheters faktisch zwei Schläuche sind, die einfach konnektiert werden können. Die Dialyseflüssigkeit tritt an einem Ende der Hohlfasermembran in diese ein und am anderen Ende wieder aus.

Der erfindungsgemäße Katheter für die Mikrodialyse weist einen sehr geringen Gesamtdurchmesser auf. Der erfindungsgemäße Katheter ist daher nicht nur sehr einfach zu implantieren und zu explantieren, er bietet auch gegenüber dem Stand der Technik einen deutlich verbesserten Tragekomfort und deutlich reduzierte Totzeiten.

Das erfindungsgemäße Verfahren zeichnet sich dadurch aus, dass die Hohlfasermembran zumindest im zu biegenden Bereich wärmebehandelt oder mit mindestens einem Lösemittel behandelt wird. Durch die anisotrope Einbringung von Wärme oder die anisotrope Behandlung mit mindestens einem Lösemittel wird die Struktur der Hohlfasermembran so verändert, dass sie bei einem minimalen Gesamtdurchmesser des Katheters etwa U-förmig gebogen werden kann, ohne abzuknicken und das Lumen zu verschließen. Dabei ist es besonders vorteilhaft, dass die Hohlfasermembran nur von außen bearbeitet werden muss, um einen erfindungsgemäßen Katheter für die Mikrodialyse herzustellen. Es zeigte sich auch, dass es nicht notwendig ist, das Lumen zu stützen, wie es z. Bsp- beim Biegen von metallischen Rohren durch Einfüllen von Sand Stand der Technik ist. Ferner ist es nicht mehr nötig, einen Schlauch oder ein anderes Teil in das Innere einer Hohlfasermembran einzuschieben oder einen Membranschlauch in ein Gehäuse einzubringen. Es besteht auch nicht mehr die Gefahr, die innere Trennschicht der Hohlfasermembran zu beschädigen.

Vorteilhafte Weiterbildungen ergeben sich aus den Unteransprüchen. Der Abstand (a) zwischen den beiden Schenkeln der Hohlfasermembran sollte vorzugsweise kleiner als 50 % des Durchmessers (d) der Hohlfasermembran sein. Alternativ oder gleichzeitig sollte der Gesamtdurchmesser des Katheters vorteilhafterweise kleiner als der 2,5fache Durchmesser der Hohlfasermembran sein. Dies bewirkt eine möglichst geringe Baugröße und einen besonders kleinen Gesamtdurchmesser des fertigen Katheters. Die Hohlfasermembran ist weiterhin im U-förmigen Bereich vorzugsweise um 180° +/- 5° gebogen und kann zur Verbesserung der Zugfestigkeit eine Verstärkung aufweisen. Durch die Verwendung einer Verstärkung wird ferner die Explantation weiter vereinfacht, da das Risiko, die Membran oder Membranteile zu verlieren, weiter verringert wird. Die Verstärkung kann bspw. ein Draht, ein Faden und/oder eine Litze sein, die bspw. parallel zu den beiden Schenkeln geführt sein kann und mit diesen verbunden, bspw. verklebt oder verschweißt ist. Die Verstärkung kann aus einem Metall, einer Metalllegierung oder einem Kunststoff bestehen. Gut geeignet sind Drähte aus Edelstahl, einem Edelmetall oder aus monofilen Polymerfasern, Fäden aus Polymerfasern oder Litzen aus mehreren dünnen metallischen Fäden.

Der erfindungsgemäße Katheter kann im Bereich des Zuflusses und/oder des Abflusses der Dialyseflüssigkeit eine flüssigkeitsimpermeable Zone aufweisen, die auch als "Schaft" für die Konnektierung der Enden dienen kann.

Die Hohlfasermembran kann ferner zumindest punktuell mindestens eine strahlungsabsorbierende Substanz, wie Farbstoffe und/oder Pigmente aufweisen, um bei der Einbringung von Wärme durch elektromagnetische Strahlung die Absorption der Strahlung und damit den Wärmeeintrag zu steigern.

Bei der Herstellung des erfindungsgemässen Katheters erfolgt die Einwirkung von Lösemitteln bevorzugt durch Aufbringen eines kleinen Tropfens eines geeigneten Lösemittels oder Lösemittelgemisches. Die Grösse des Tropfens richtet sich nach dem Durchmesser und der Wanddicke der Hohlfasermembran. Das Volumen sollte ausreichen, um die Porenstruktur der Membran auf einer Länge entsprechend dem ein- bis eineinhalbfachen Durchmesser und ca. 50 % des Membranumfangs aufzufüllen. Geeignete Lösemittel sind solche, die das Polymer der Membran lösen oder erweichen und zum Schrumpfen bringen, z.B. Dimethylformamid, Dimethylsulfoxid, Dimethylacetamid oder Tetrahydrofuran. Chlorierte Kohlenwasserstoffe z.B. Trichlorethen sind aus Umweltgründen nicht bevorzugt. Besonders bevorzugt sind Lösemittel oder Lösemittelgemische, die durch Verdampfen leicht wieder entfernt werden können, z.B. Gemische aus Isopropanol und Tetrahydrofuran.

Bei der Herstellung des erfindungsgemäßen Katheters hat sich die Einbringung von Wärme über den direkten Kontakt mit einem elektrisch aufgeheizten Draht als besonders geeignet erwiesen. Die Einbringung von Wärme durch elektromagnetische Strahlung ist auch möglich, bspw. durch Laserstrahlung, Mikrowellenstrahlung oder Hochfrequenzstrahlung, unter der Voraussetzung, dass die Strahlung von der Hohlfasermembran zumindest im gebogenen Bereich absorbiert wird.

Als Hohlfasermembran sind handelsübliche Dialysehohlfasern, bspw. aus Polyamiden, Polyamid S, Polyarylethersulfonen, Polymethacrylat, Polysulfonen oder Polycarbonat-Polyether-Blockcopolymeren (bspw. unter dem Handelsnamen Gambrane erhältlich) geeignet. Besonders geeignet sind Hohlfasermembranen mit einer asymmetrischen Struktur, dergestalt, dass das Lumen von einer feinporigen Trennschicht umgeben ist, während die Wandung nach außen zunehmend größere und/oder offene Poren aufweist.

Der Außendurchmesser der Hohlfasermembran sollte 600 µm nicht überschreiten, besser noch unter 300 µm und besonders bevorzugt unter 200 µm liegen. Geeignet sind auch Hohlfasermembranen mit kleinem Durchmesser, bspw. einem Innendurchmesser im Bereich von 50 bis 100 µm, vorzugsweise 60 bis 90 µm und geringer Wandstärke, bspw. 20 bis 80 µm, vorzugsweise 40 µm. Letztere können zu erfindungsgemäßen Kathetern mit besonders geringem Gesamtdurchmesser verarbeitet werden.

Die Verklebung mit der Verstärkung kann durch Aufgabe einer dünnen Kleberschicht auf die Verstärkung und anschließende Verbindung mit der Hohlfasermembran erfolgen. Als Kleber eignen sich Reaktivkleber wie Cyanacrylatkleber oder Polyurethankleber sowie lösemittelhaltige Kleber oder durch Strahlung aktivierbare Kleber oder thermisch aktivierbare Polymere oder Kleber (Schmelzkleber) wie Ethylen/Ethylacrylat-Copolymere, Ethylen/Vinylacrylat-Copolymere, Polyamide, Polyester, Polyisobutylen oder Polyvinylbutyrate sowie die Membranmaterialien selbst. Es ist auch möglich, die Verstärkung direkt mit der Membran zu verbinden ("Bonden"), indem die Hohlfasermembran in innigen Kontakt mit der Verstärkung gebracht und die Verstärkung auf eine Temperatur oberhalb der Schmelztemperatur des Membranmaterials und/oder, bei Verwendung eines Verstärkers aus Kunststoff, des Verstärkermaterials gebracht wird. Dieses Verfahren eignet sich auch zur Aktivierung eines thermisch aktivierbaren Klebers. Wenn ein metallischer Verstärker verwendet wird, kann die Erwärmung durch Zuführung elektrischer Energie über den Verstärker erfolgen. Dieses Verfahren ist besonders bevorzugt, da überraschenderweise gefunden wurde, dass beim Aufschmelzen nur die äußere großporige und/oder offenporige Kontaktzone zwischen Membran und Verstärkung verdichtet wird, der zugehörige Bereich der Trennschicht jedoch nicht verändert wird und durchgängig bleibt. Damit wird die Austauschleistung durch diese Art der Verklebung zwischen der Hohlfasermembran und der Verstärkung nur minimal reduziert.

Zur Sicherung der Verstärkung des erfindungsgemäßen Katheters kann zusätzlich ein Tropfen eines reaktiven Polymergemisches am vorderen, d.h. am gebogenen Ende des Katheters aufgebracht werden, welcher sowohl die Haftung zwischen Membran und Verstärkung weiter verbessert als auch eventuell vorhandene Schwachstellen und Lecks in der Membran im gebogenen Bereich verschließt. Dazu können auch die bereits genannten Klebstoffe verwendet werden.

Die flüssigkeitsimpermeable Zone im Bereich des Zuflusses und/oder Abflusses des erfindungsgemäßen Katheters kann bspw. durch Tränken der Hohlfasermembran mit einem geeigneten Polymer oder einem Polymergemisch, bspw. einem Kleber, geschaffen werden, welches dann thermisch oder reaktiv ausgehärtet werden kann. Bevorzugt ist aber, diese Bereiche bis in die Nähe des Schmelzpunktes des Membranmaterials zu erhitzen, so dass die Poren in der Hohlfasermembran kollabieren und die Membranwandung auf diese Weise praktisch flüssigkeitsundurchlässig wird. Damit geht eine Schrumpfung der Hohlfasermembran einher, was neben der Reduzierung der Wandstärke auch eine Reduzierung des Innendurchmessers zur Folge hat. Der Vorteil dieser Vorgehensweise besteht darin, dass durch die Reduzierung des Innendurchmessers der Hohlfasermembran das Totvolumen des erfindungsgemäßen Katheters reduziert wird. Dadurch wiederum wird die Verweilzeit einer Probe im Bereich des Zuflusses und/oder Abflusses reduziert und eine Messgröße, bspw. eine Änderung des Glucosegehalts im Gewebe, kann schneller detektiert werden.

Die thermische Behandlung im Bereich des Zuflusses und/oder Abflusses kann durch Einwirkung von Heißluft, durch partielles Einbringen des Katheters in eine heiße Kammer oder zwischen zwei erhitzte Backen oder durch Einwirkung von Strahlung erfolgen. Bspw. IR-Strahlung eignet sich besonders gut zur präzisen räumlichen Formung des Katheters, wenn dafür gesorgt wird, dass nur in den aufzuheizenden Segmenten eine Absorption der Strahlung stattfindet. Dies kann durch Zugabe geeigneter strahlungsabsorbierender Substanzen wie Farbstoffe oder Pigmente erfolgen.

Die Ausführung des Zuflusses und/oder Abflusses des erfindungsgemäßen Katheters wird so gewählt, dass die beiden Enden der Hohlfasermembran getrennt voneinander vorliegen. Sie können dann in an sich bekannter Weise in Ausnehmungen in einem Schlauch oder in einer Trägerplatte mit miniaturisierten Fließwegen eingelegt und dann eingeklebt werden. Die Verstärkung kann dann in oder an einem Schlauch oder einer Trägerplatte mit miniaturisierten Fließwegen, bspw. in einer Ausnehmung oder an einer Fläche der Trägerplatte in an sich bekannter Weise befestigt, z. Bsp. eingeklebt werden.

Im Folgenden wird ein Ausführungsbeispiel der vorliegenden Erfindung näher erläutert. Es zeigen:
- Figur 1: eine schematische, nicht maßstabsgetreue Darstellung einer Hohlfasermembran für einen erfindungsgemäßen Katheter nach dem Biegen;

- Figur 2a: den hinteren Teil einer Hohlfasermembran aus Figur 1 mit einer Verstärkung;
- Figur 2b: die Hohlfasermembran aus Figur 1 mit einer Verstärkung in ihrer Gesamtheit;
- Figur 3: einen schematischen Querschnitt durch die Hohlfasermembran aus den Figuren 2a und 2b.

### 1a. Biegen der Hohlfasermembran

Eine PAS-Hohlfasermembran der Fa. Gambro Dialysatoren, Hechingen, Deutschland, mit einem Innendurchmesser von 214 µm und einer Wandstärke von 43 µm wird auf eine Länge von 50 mm geschnitten. Das Hohlfasermembranstück wird mittig auf einen elektrisch beheizbaren Konstantandraht mit einem Durchmesser von 250 µm gelegt. Die beiden Enden der Hohlfasermembran werden mit einer Kraft von 1 mg belastet. Dieser Draht wird elektrisch beheizt. Aus einem stromgeregelten Netzteil fließt über einen Zeitraum von 2x2 Sekunden ein Strom von 1,7 A. Unter dem Einfluss des erhitzten Drahtes verformt sich die Hohlfasermembran an der Auflagestelle und biegt sich. Dabei bilden die beiden Schenkel einen Winkel von 15° bis 30°. Durch vorsichtiges Zusammendrücken im Augenblick des Abschalten des Stromes im zweiten Zyklus kann der Winkel auf praktisch Null reduziert werden.

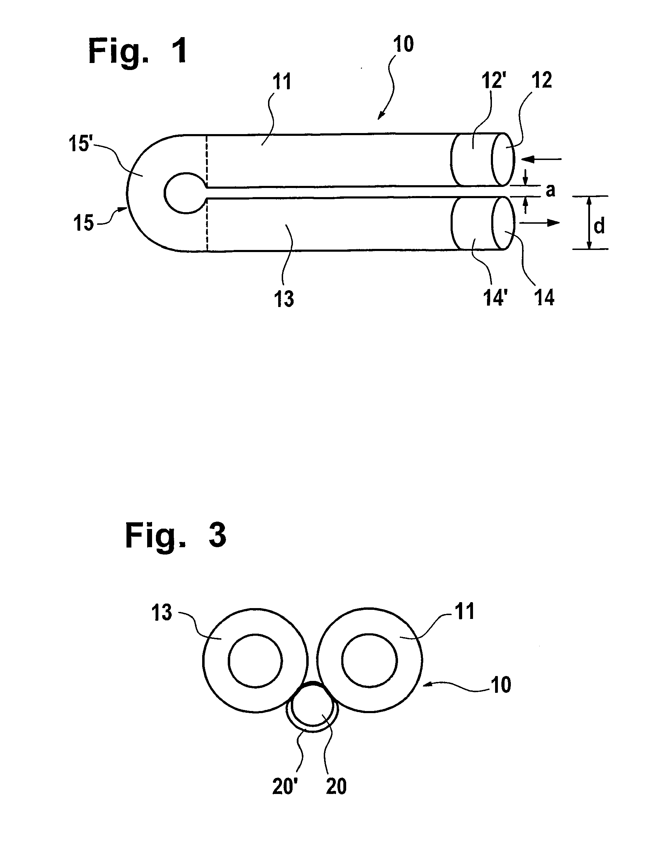

Figur 1 zeigt schematisch eine solche U-förmig gebogene Hohlfasermembran 10 mit zwei Schenkeln 11, 13, die zu je einem Zufluss 12 bzw. Abfluss 14 münden und einem gebogenen Bereich 15. Die Fließrichtung der Dialyseflüssigkeit ist mit Pfeilen angedeutet. Der Abstand a zwischen den Schenkeln 11, 13 beträgt weniger als 50 % des Durchmessers d der Hohlfasermembran 10. Im Bereich des Zuflusses 12 und des Abflusses 14 befindet sich je ein flüssigkeitsimpermeabler Bereich 12', 14'. Ein weiterer flüssigkeitsimpermeabler Bereich 15' befindet sich im gebogenen Bereich 15, welcher mit einem Klebstoff getränkt ist.

### 1b. Teilautomatisches Biegen einer Hohlfasermembran

Eine PAS-Hohlfasermembran der Fa. Gambro Dialysatoren, Hechingen, Deutschland, mit einem Innendurchmesser von 214 µm und einer Wandstärke von 43 µm wird auf eine Länge von 50 mm geschnitten und auf eine Auflage gelegt. Die Auflage hat in der Mitte eine schmale, gerade Ausnehmung, in der ein senkrecht zur Oberfläche orientierter, elektrisch beheizbarer Konstantandraht mit einem Durchmesser von 250 µm in einer gleichförmigen Bewegung mit ca. 10 mm/s geführt wird. Durch den Draht fließt ein Strom von .1,7 A. Bei der Vorwärtsbewegung trifft der Draht mittig auf die Hohlfasermembran und schleppt sie mit. Seitlich des Verfahrweges des Drahtes befinden sich Wandungen, deren Abstand vom Pfad des Drahtes sich asymptotisch auf 500 µm verringert. Unter Einfluss des durch den Stromfluss beheizten Drahtes und der Wandungen biegt sich die mitgeschleppte Hohlfaser in gewünschter Weise. Durch das Zusammenfahren zweier seitlicher Backen aus Silikon im Augenblick des Abschaltens des Stromes kann der Winkel zwischen den beiden Schenkeln auf praktisch Null reduziert werden. Die Bewegungen des Drahtes und der Backen und der Stromfluss werden automatisch gesteuert. Damit ist eine reproduzierbare Ausformung der Biegung problemlos möglich.

### 2. Herstellung eines Verstärkungsdrahtes

Zur Verstärkung wird ein Draht aus rostfreiem Stahl (Werkstoff-Nr. 1.4301, Fe/Cr18/Ni10, Härtegrad: geglüht, Durchmesser: 0,05 mm) der Fa. Goodfellow Deutschland, Bad Nauheim, Deutschland, mit der Bestellnummer FE225110 eingesetzt. Zur Verbesserung der Haftung wird der Draht mit einer dünnen Schicht aus einem Polyamid überzogen. Dazu wird der Draht mit einer Geschwindigkeit von 3,1 m/min durch eine Beschichtdüse (Durchmesser 300 µm) gezogen und allseitig mit einer Schicht einer Lösung aus 15 Gew.-% Trogamid T3000, Hersteller Creanova, Marl, Deutschland, 7 Gew.-% PVP Plasdone C-15, Hersteller ISP Technologies, Inc., Wayne, USA und 78 Gew.-% NMP (N-methyl-2-pyrrolidon), Hersteller Merck, Darmstadt, Deutschland, versehen. Der so beschichtete Draht wird durch ein Wasserbad gezogen, um das Lösemittel auszuwaschen. Es bildet sich eine feste weiße Schicht mit einer Dicke von ca. 100 µm. Nach dem Trocknen an Luft ist der beschichtete Verstärkungsdraht einsetzbar.

### 3. Verstärken der Hohlfasermembran

Eine wie unter 1a. oder 1b. behandelte Hohlfasermembran wird so um einen wie unter 2 behandelten, praktisch endlosen Verstärkungsdraht gewickelt, dass die beiden Schenkel der Hohlfasermembran dicht aneinander liegen und gemeinsam eine Windung mit einer Steigung von 5 bis 10 mm pro Windung bilden. Die Biegung wird dabei direkt auf den Draht gedrückt, während Anfang und Ende der Hohlfasermembran auf der Länge von je ca. 3 mm vom Draht abstehen. Durch den Draht wird über eine Dauer von 3,5 Sekunden ein elektrischer Strom von 0,2 A geleitet. Der durch den Strom erhitzte Draht schmilzt die Beschichtung des Verstärkungsdrahtes und die Hohlfasermembran im Außenbereich auf. Nach Abschalten des Stroms kühlt der Draht ab, und die Hohlfasermembran haftet fest.

### 4. Fertigstellung des Katheters

Die wie unter 1a. oder 1b. und 3. behandelte Hohlfasermembran wird vereinzelt. Dazu wird der Verstärkungsdraht unmittelbar vor der Biegung abgeschnitten und auf ca. 30 mm abgelängt. Die beiden offenen Enden der Hohlfasermembran werden in einen Schlauch mit einem Innendurchmesser von 500 µm eingeklebt. Als Klebstoff dient Epoxidharz UHU plus schnellfest, UHU Vertrieb GmbH, Bühl/Baden, Deutschland. Die beiden Enden der Hohlfasermembran werden auf einer Länge von ca. 4 mm mit Klebstoff getränkt und damit impermeabel gemacht. Als Klebstoff dient UV-härtendes Dymax 1181-M, Dymax Europe GmbH, Frankfurt a.M., Deutschland. Als permeable Austauschlänge verbleiben 2x20 mm.

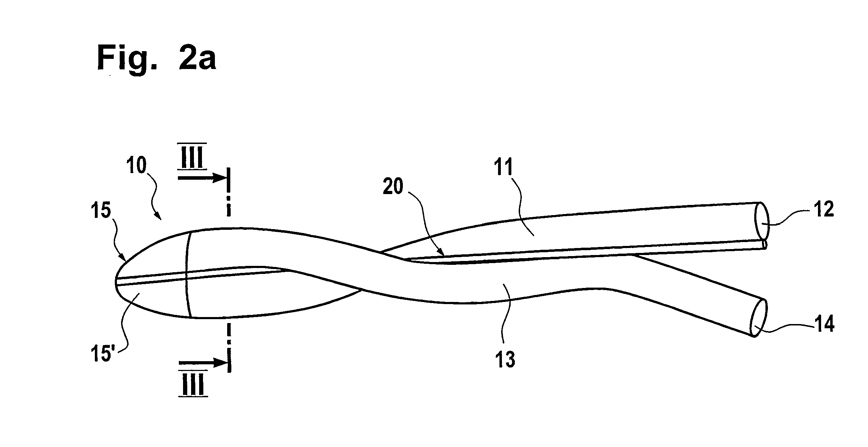

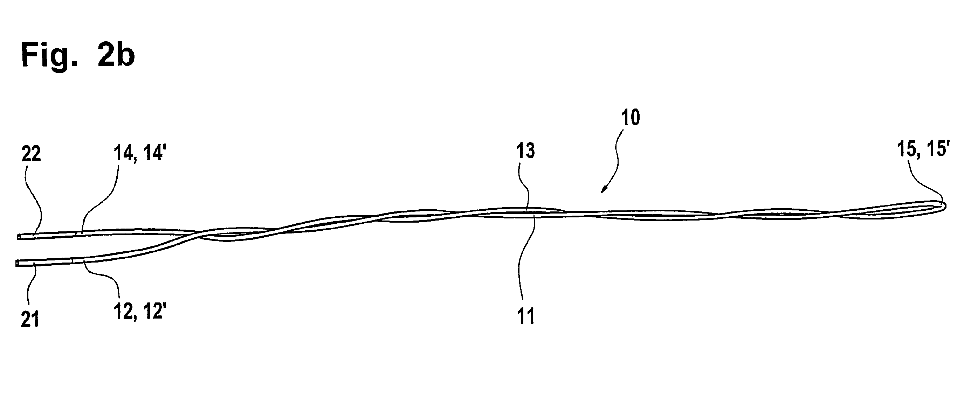

Die Figuren 2a und 2b zeigen schematisch eine wie unter 3. verstärkte Hohlfasermembran 10, die um einen Verstärkungsdraht 20 gewickelt ist. Der gebogene Bereich 15 ist mit dem Verstärkungsdraht 20 verbunden, während Zufluss 12 und Abfluss 14 bspw. über die Länge der impermeablen Bereiche 12' und 14' vom Verstärkungsdraht 20 abstehen. Angedeutet sind ferner Schläuche 21, 22, in welche der Zufluss 12 und der Abfluss 14 eingeklebt sind.

Figur 3 zeigt einen Querschnitt entlang der Linie III-III in Figur 2a. Man erkennt, dass die Beschichtung 20' des Verstärkungsdrahts 20 aufgeschmolzen und mit dem Außenbereich der Hohlfasermembran verschmolzen ist.

### 5. Messung der Stoffaustauschleistung

Ein wie unter 4. hergestellter Katheter wird mit destilliertem Wasser mit einem Fluss von 0,1 µl/min durchströmt und in ein Becherglas mit 200 mg/dl Glucose eingebracht. Am Ausgang des Katheters wird ein Glucosegehalt in der Dialyseflüssigkeit von 199 mg/dl gemessen.

### 6. Messung der Totzeit (95)

Der Begriff Totzeit (95) bezeichnet die Totzeit bis zum Erreichen einer Signaländerung von 95%. Ein wie unter 4. hergestellter Katheter wird mit einer geeigneten Durchflussbrechungsindexmesszelle kombiniert und mit destilliertem Wasser mit einem Fluss von 0,1 µl/min durchströmt. Der Katheter wird wechselweise in Bechergläser mit 200 mg/dl Glucose und mit destilliertem Wasser eingebracht. Die Glucosekonzentration in der Messzelle folgt der Konzentration im Becherglas mit Zeitverzögerung. Nach Subtraktion der durch das Volumen der Messzelle und ihrer Zuleitung verursachten Verzögerung verbleiben für die Totzeit(95) des Katheters 252 Sekunden.

## Patentansprüche

1. Katheter mit einer im wesentlichen U-förmig gebogenen Hohlfasermembran (10), mit zwei Schenkeln (11, 13) und einem gebogenen Bereich (15), **dadurch gekennzeichnet,**
**dass** der Abstand (a) zwischen den beiden Schenkeln (11, 13) kleiner als 50 % des Durchmessers (d) der Hohlfasermembran (10) ist.

2. Katheter nach Anspruch 1, **dadurch gekennzeichnet,**
**dass** der Gesamtdurchmesser des Katheters kleiner als der 2,5fache Durchmesser der Hohlfasermembran ist.

3. Katheter nach Anspruch 1 oder 2, **dadurch gekennzeichnet,**
**dass** die Hohlfasermembran (10) im U-förmigen Bereich um 180° +/- 5° gebogen ist.

4. Katheter nach einem der vorhergehenden Ansprüche, **dadurch gekennzeichnet,**
**dass** die Hohlfasermembran (10) eine Verstärkung (20) aufweist.

5. Katheter nach Anspruch 4, **dadurch gekennzeichnet,**
**dass** als Verstärkung (20) ein Draht, ein Faden und/oder eine Litze vorgesehen und mit den beiden Schenkeln (11, 13) verbunden, bspw. verklebt oder verschweißt ist.

6. Katheter nach Anspruch 4 oder 5, **dadurch gekennzeichnet,**
**dass** die Verstärkung (20) aus einem Metall, einer Metalllegierung oder einem Kunststoff besteht.

7. Katheter nach Anspruch 5 oder 6, **dadurch gekennzeichnet,**
**dass** der Draht aus Edelstahl, einem Edelmetall oder aus monofilen Polymerfasern besteht.

8. Katheter nach Anspruch 5 oder 6, **dadurch gekennzeichnet,**
**dass** der Faden aus Polymerfasern besteht.

9. Katheter nach Anspruch 5 oder 6, **dadurch gekennzeichnet,**
**dass** die Litze aus mehreren dünnen metallischen Fäden besteht.

10. Katheter nach einem der Ansprüche 5 bis 9, **dadurch gekennzeichnet,**
**dass** die Hohlfasermembran (10) zumindest punktuell mindestens eine strahlungsabsorbierende Substanz, vorzugsweise Farbstoffe und/oder Pigmente aufweist.

11. Katheter nach einem der vorhergehenden Ansprüche, **dadurch gekennzeichnet,**
**dass** im Bereich des Zuflusses (12) und/oder Abflusses (14) der Dialyseflüssigkeit eine flüssigkeitsimpermeable Zone (12', 14') vorgesehen ist.

12. Katheter nach einem der vorhergehenden Ansprüche, **dadurch gekennzeichnet,**
**dass** die Hohlfasermembran eine asymmetrische Struktur aufweist, dergestalt, dass das Lumen von einer feinporigen Trennschicht umgeben ist, während die Wandung nach außen zunehmend größere Poren aufweist.

13. Katheter nach einem der vorhergehenden Ansprüche, **dadurch gekennzeichnet,**
**dass** der Außendurchmesser der Hohlfasermembran (10) kleiner als 600 µm, bevorzugt kleiner als 300 µm, besonders bevorzugt kleiner als 200 µm ist.

14. Katheter nach einem der vorhergehenden Ansprüche, **dadurch gekennzeichnet,**
**dass** der Innendurchmesser der Hohlfasermembran (10) 50 bis 100 µm, vorzugsweise 60 bis 90 µm beträgt.

15. Katheter nach einem der vorhergehenden Ansprüche, **dadurch gekennzeichnet,**
**dass** die Wandstärke der Hohlfasermembran (10) 20 bis 80 µm, vorzugsweise 40 µm beträgt.

16. Verfahren zur Herstellung eines Katheters mit einer im wesentlichen U-förmig gebogenen Hohlfasermembran (10) nach Patentanspruch 1,
**dadurch gekennzeichnet,**
**dass** die Hohlfasermembran (10) zumindest im zu biegenden Bereich wärmebehandelt und/oder mit mindestens einem Lösemittel behandelt wird.

17. Verfahren nach Anspruch 16, **dadurch gekennzeichnet,**
**dass** die Hohlfasermembran über einen direkten Kontakt mit einem elektrisch aufgeheizten Draht und/oder durch Einwirkung von elektro-magnetischer Strahlung behandelt wird.

18. Verfahren nach einem der Ansprüche 16 oder 17, **dadurch gekennzeichnet,**
**dass** handelsübliche Dialysehohlfasern verwendet werden.

19. Verfahren nach einem der Ansprüche 16 oder 17, **dadurch gekennzeichnet,**
**dass** Dialysehohlfasern mit kleinerem Durchmesser und/oder geringerer Wandstärke als handelsübliche Dialysehohlfasern verwendet werden.

20. Verfahren nach einem der Ansprüche 16 bis 19, **dadurch gekennzeichnet,**
**dass** zum Verkleben einer Verstärkung (20) zunächst eine dünne Kleberschicht auf die Verstärkung (20) aufgetragen und diese dann mit der Hohlfasermembran (10) verbunden wird.

21. Verfahren nach Anspruch 20, **dadurch gekennzeichnet,**
**dass** zum Verkleben Reaktivkleber, lösemittelhaltige Kleber, durch Strahlung aktivierbare Polymere oder Kleber oder thermisch aktivierbare Kleber (Schmelzkleber) verwendet werden.

22. Verfahren nach einem der Ansprüche 16 bis 19, **dadurch gekennzeichnet,**
**dass** die Verstärkung (20) direkt mit der Hohlfasermembran (10) verbunden wird.

23. Verfahren nach Anspruch 22, **dadurch gekennzeichnet,**
**dass** die Hohlfasermembran (10) in innigen Kontakt mit der Verstärkung (20) gebracht und die Verstärkung auf eine Temperatur oberhalb der Schmelztemperatur des Membranmaterials und/oder des Verstärkermaterials gebracht wird.

24. Verfahren nach einem der Ansprüche 16 bis 23, **dadurch gekennzeichnet,**
**dass** am vorderen Ende der Hohlfasermembran (10) ein Tropfen eines reaktiven Polymergemisches aufgebracht wird.

25. Verfahren nach einem der Ansprüche 16 bis 24, **dadurch gekennzeichnet,**
**dass** die Hohlfasermembran (10) im Bereich des Zuflusses (12) und/oder Abflusses (14) des Katheters mit mindestens einem härtbaren Polymer getränkt wird und das Polymer anchließend gehärtet wird.

26. Verfahren nach einem der Ansprüche 16 bis 24, **dadurch gekennzeichnet,**
**dass** die Hohlfasermembran (10) im Bereich des Zuflusses (12) und/oder Abflusses (14) des Katheters bis in die Nähe des Schmelzpunktes des Membranmaterials erhitzt wird.

27. Verfahren nach Anspruch 26, **dadurch gekennzeichnet,**
**dass** die Hohlfasermembran (10) durch Einwirkung von Strahlung, vorzugsweise IR-Strahlung erhitzt wird.

28. Verfahren nach einem der Ansprüche 16 bis 27, **dadurch gekennzeichnet,**
**dass** die Enden der Hohlfasermembran (10) in einen Schlauch oder eine Trägerplatte mit miniaturisierten Fließwegen eingelegt und dann eingeklebt werden.

29. Verfahren nach Anspruch 28, **dadurch gekennzeichnet,**
**dass** die Verstärkung (20) in oder an einem Schlauch oder einer Trägerplatte mit miniaturisierten Fließwegen befestigt, bspw. eingeklebt wird.

## Claims

1. Catheter with a hollow fiber membrane (10) bent essentially in a U-shape comprising two legs (11, 13) and a bent section (15), **characterized in that** the distance (a) between the two legs (11, 13) is less than 50 % of the diameter (d) of the hollow fiber membrane (10).

2. Catheter as claimed in claim 1, **characterized in that** the overall diameter of the catheter is less than 2.5 times the diameter of the hollow fiber membrane.

3. Catheter as claimed in claim 1 or 2, **characterized in that** the U-shaped section of the hollow fiber membrane (10) is bent by 180° ± 5°.

4. Catheter as claimed in one of the previous claims, **characterized in that** the hollow fiber membrane (10) has a reinforcement (20).

5. Catheter as claimed in claim 4, **characterized in that** a wire, thread and/or braid are provided as the reinforcement (20) which is bonded to the two legs (11, 13) by for example gluing or welding.

6. Catheter as claimed in claim 4 or 5, **characterized in that** the reinforcement (20) consists of a metal, a metal alloy or a plastic.

7. Catheter as claimed in claim 5 or 6, **characterized in that** the wire consists of high-grade steel, a noble metal or monofil polymer fibers.

8. Catheter as claimed in claim 5 or 6, **characterized in that** the thread consists of polymer fibers.

9. Catheter as claimed in claim 5 or 6, **characterized in that** the braid consists of several thin metallic threads.

10. Catheter as claimed in one of the claims 5 to 9, **characterized in that** at least a particular site on the hollow fiber membrane (10) has at least one substance that absorbs radiation preferably dyes and/or pigments.

11. Catheter as claimed in one of the previous claims, **characterized in that** a liquid-impermeable zone (12', 14') is provided in the area of the inlet (12) and/or outlet (14) for the dialysis fluid.

12. Catheter as claimed in one of the previous claims, **characterized in that** the hollow fiber membrane has an asymmetric structure such that the lumen is surrounded by a fine-pored separation layer whereas the wall has pores which increase in size towards the outside.

13. Catheter as claimed in one of the previous claims, **characterized in that** the external diameter of the hollow fiber membrane (10) is less than 600 µm, preferably less than 300 µm and particularly preferably less than 200 µm.

14. Catheter as claimed in one of the previous claims, **characterized in that** the internal diameter of the hollow fiber membrane (10) is 50 to 100 µm, preferably 60 to 90 µm.

15. Catheter as claimed in one of the previous claims, **characterized in that** the wall thickness of the hollow fiber membrane (10) is 20 to 80 µm, preferably 40 µm.

16. Method for producing a catheter with a hollow fiber membrane (10) which is essentially bent in a U-shape or for producing a hollow fiber membrane which is essentially bent in a U-shape, **characterized in that** the hollow fiber membrane (10) is heat-treated and/or treated with at least one solvent at least in the region to be bent.

17. Method as claimed in claim 16, **characterized in that** the hollow fiber membrane is treated by direct contact with an electrically heated wire and/or by the action of electromagnetic radiation.

18. Method as claimed in one of the claims 16 or 17, **characterized in that** commercial hollow dialysis fibers are used.

19. Method as claimed in one of the claims 16 or 17, **characterized in that** hollow dialysis fibers having a smaller diameter and/or smaller wall thickness than commercial hollow dialysis fibers are used.

20. Method as claimed in one of the claims 16 to 19, **characterized in that** in order to bond a reinforcement (20), firstly a thin layer of adhesive is applied to the reinforcement (20) which is then bonded to the hollow fiber membrane (10).

21. Method as claimed in claim 20, **characterized in that** reactive adhesives, solvent-containing adhesives, polymers or adhesives that can be activated by radiation or thermally activatable adhesives (hot-melt adhesives) are used for the bonding.

22. Method as claimed in one of the claims 16 to 19, **characterized in that** the reinforcement (20) is bonded directly to the hollow fiber membrane (10).

23. Method as claimed in claim 22, **characterized in that** the hollow fiber membrane (10) is intimately contacted with the reinforcement (20) and the reinforcement is heated to a temperature above the melting temperature of the membrane material and/or above that of the reinforcement material.

24. Method as claimed in one of the claims 16 to 23, **characterized in that** a drop of a reactive polymer mixture is applied to the front end of the hollow fiber membrane (10).

25. Method as claimed in one of the claims 16 to 24, **characterized in that** the hollow fiber membrane (10) in the area of the inlet (12) and/or outlet (14) of the catheter is impregnated with a hardenable polymer and the polymer is subsequently hardened.

26. Method as claimed in one of the claims 16 to 24, **characterized in that** the hollow fiber membrane (10) in the area of the inlet (12) and/or outlet (14) of the catheter is heated to near the melting point of the membrane material.

27. Method as claimed in claim 26, **characterized in that** the hollow fiber membrane (10) is heated by the action of radiation, preferably IR radiation.

28. Method as claimed in one of the claims 16 to 27, **characterized in that** the ends of the hollow fiber membrane (10) are inserted into a tube or a carrier plate with miniaturized flow paths and then glued in.

29. Method as claimed in claim 28, **characterized in that** the reinforcement (20) is attached or glued in or on a tube or a carrier plate with miniaturized flow paths.

## Revendications

1. Cathéter comprenant une membrane en fibres creuses (10) pliée essentiellement en forme de U, comprenant deux branches (11, 13) et une zone courbe (15), **caractérisé en ce que** la distance (a) entre les deux branches (11, 13) représente moins de 50 % du diamètre (d) de la membrane en fibres creuses (10).

2. Cathéter selon la revendication 1, **caractérisé en ce que** le diamètre total du cathéter est inférieur à 2,5 fois le diamètre de la membrane en fibres creuses.

3. Cathéter selon la revendication 1 ou 2, **caractérisé en ce que** la membrane en fibres creuses (10) est pliée en formant un angle de 180° ± 5° dans la zone en forme de U.

4. Cathéter selon l'une quelconque des revendications précédentes, **caractérisé en ce que** la membrane en fibres creuses (10) présente un renforcement (20).

5. Cathéter selon la revendication 4, **caractérisé en ce qu'**on prévoit, à titre de renforcement (20), un fil métallique, un brin et/ou un cordon, ledit renforcement étant relié, par exemple collé ou soudé, aux deux branches (11, 13).

6. Cathéter selon la revendication 4 ou 5, **caractérisé en ce que** le renforcement (20) est constitué d'un métal, d'un alliage métallique ou d'une matière synthétique.

7. Cathéter selon la revendication 5 ou 6, **caractérisé en ce que** le fil métallique est constitué d'acier inoxydable, d'un métal noble ou de fibres polymères à monofilaments.

8. Cathéter selon la revendication 5 ou 6, **caractérisé en ce que** le brin est constitué par des fibres polymères.

9. Cathéter selon la revendication 5 ou 6, **caractérisé en ce que** le cordon est constitué par plusieurs minces brins métalliques.

10. Cathéter selon l'une quelconque des revendications 5 à 9, **caractérisé en ce que** la membrane en fibres creuses (10) présente, tout au moins ponctuellement, au moins une substance absorbant les rayonnements, de préférence des colorants et/ou des pigments.

11. Cathéter selon l'une quelconque des revendications précédentes, **caractérisé en ce que**, dans la zone de l'afflux (12) et/ou de l'évacuation (14) du liquide de dialyse, on prévoit une zone imperméable aux liquides (12', 14').

12. Cathéter selon l'une quelconque des revendications précédentes, **caractérisé en ce que** la membrane en fibres creuses présente une structure asymétrique de telle sorte que la lumière est entourée d'une couche de séparation finement poreuse, tandis que la paroi présente des pores dont la dimension augmente vers l'extérieur.

13. Cathéter selon l'une quelconque des revendications précédentes, **caractérisé en ce que** le diamètre externe de la membrane en fibres creuses (10) est inférieur à 600 µm, de préférence inférieur à 300 µm, de manière particulièrement préférée inférieur à 200 µm.

14. Cathéter selon l'une quelconque des revendications précédentes, **caractérisé en ce que** le diamètre interne de la membrane en fibres creuses (10) s'élève de 50 à 100 µm, de préférence de 60 à 90 µm.

15. Cathéter selon l'une quelconque des revendications précédentes, **caractérisé en ce que** l'épaisseur de paroi de la membrane en fibres creuses (10) s'élève de 20 à 80 µm, de préférence à 40 µm.

16. Procédé pour la fabrication d'un cathéter possédant une membrane en fibres creuses (10) pliée essentiellement en forme de U, selon la revendication 1, **caractérisé en ce que**, tout au moins dans la zone qui doit être pliée, on soumet la membrane en fibres creuses (10) à un traitement thermique et/ou on la traite avec au moins un solvant.

17. Procédé selon la revendication 16, **caractérisé en ce qu'**on traite la membrane en fibres creuses par une mise en contact directe avec un fil chauffé par voie électrique et/ou par exposition à un rayonnement électromagnétique.

18. Procédé selon l'une quelconque des revendications 16 ou 17, **caractérisé en ce qu'**on utilise des fibres creuses pour dialyse disponibles dans le commerce.

19. Procédé selon l'une quelconque des revendications 16 ou 17, **caractérisé en ce qu'**on utilise des fibres creuses pour dialyse possédant un diamètre inférieur et/ou une épaisseur de paroi inférieure à celui ou à celle de fibres creuses pour dialyse disponibles dans le commerce.

20. Procédé selon l'une quelconque des revendications 16 à 19, **caractérisé en ce que**, pour le collage d'un renforcement (20), on applique d'abord une mince couche adhésive sur le renforcement (20) et on relie ensuite ce dernier à la membrane en fibres creuses (10).

21. Procédé selon la revendication 20, **caractérisé en ce qu'**on utilise pour le collage, des adhésifs réactifs, des adhésifs contenant des solvants, des polymères ou des adhésifs activables par rayonnement ou encore des adhésifs activables par voie thermique (des adhésifs thermofusibles).

22. Procédé selon l'une quelconque des revendications 16 à 19, **caractérisé en ce que** le renforcement (20) est relié directement à la membrane en fibres creuses (10).

23. Procédé selon la revendication 22, **caractérisé en ce que** la membrane en fibres creuses (10) est amenée en contact intime avec le renforcement (20) et le renforcement est amené à une température supérieure à la température de fusion de la matière de la membrane et/ou de la matière du renforcement.

24. Procédé selon l'une quelconque des revendications 16 à 23, **caractérisé en ce qu'**on applique, sur l'extrémité avant de la membrane en fibres creuses (10), une goutte d'un mélange polymère réactif.

25. Procédé selon l'une quelconque des revendications 16 à 24, **caractérisé en ce qu'**on imprègne la membrane en fibres creuses (10) dans la zone de l'afflux (12) et/ou ou de l'évacuation (14) du cathéter avec au moins un polymère durcissable et on durcit ensuite le polymère.

26. Procédé selon l'une quelconque des revendications 16 à 24, **caractérisé en ce qu'**on chauffe la membrane en fibres creuses (10) dans la zone de l'afflux (12) et/ou de l'évacuation (14) du cathéter jusqu'à proximité du point de fusion de la matière de la membrane.

27. Procédé selon la revendication 26, **caractérisé en ce que** la membrane en fibres creuses (10) est chauffée par exposition à un rayonnement, de préférence un rayonnement infrarouge.

28. Procédé selon l'une quelconque des revendications 16 à 27, **caractérisé en ce qu'**on introduit les extrémités de la membrane en fibres creuses (10) dans un tuyau flexible ou dans une plaque de support comprenant des voies d'écoulement miniaturisées et on les y colle.

29. Procédé selon la revendication 28, **caractérisé en ce qu'**on fixe, par exemple par collage le renforcement 20 dans ou sur un tuyau flexible ou bien dans ou sur une plaque de support comprenant des voies d'écoulement miniaturisées.
